# EUROPEAN PATENT APPLICATION

(11) **EP 0 712 634 A2**
(43) Date of publication of application: **22.05.1996**
(21) Application number: 95500155.7
(22) Date of filing: 14.11.1995
(51) Int. Cl.: A61L 11/00, A61L 2/18

(54) **Procedure, formula and equipment for the treatment and sterilization of biological, solid, liquid, iron-based metallic, non iron-based metallic, toxic and dangerous hospital residuals**

(30) Priority: 15.11.1994 ES 9402346
(71) Applicant: Berlanga Barrera, Manuel, ES-46008 Valencia (ES)
(72) Inventor: Berlanga Barrera, Manuel, ES-46008 Valencia (ES)
(74) Representative: Ungria Lopez, Javier

(57) **Abstract**

Procedure, formula and equipment for the treatment and sterilization of biological, solid, liquid, iron-based metallic, non iron-based metallic, toxic and dangerous hospital residuals. It consists of the formula : O₂ (oxygen)+ O₃ (ozone) + H₂O (water) // O₂ (oxygen) + O₃ (ozone) + CO₂ (carbon dioxide) + H₂O (water) in variable proportions and volumes which comes into the equipment from the oxygen bottle (10), the carbon dioxide bottle (11), which are connected to an ozonizer (1) through conducts (13) and (15). This ozonizer has a water refrigeration entrance and a water refrigeration exit (16) and (17). The conducts (13) and (15) run parallel to the ozone conduct (14) to the injector (3) which is triggered by the pump (2) for the passing through the mixing conduct (4) to the absorption tank (5), from where a homogenous flow of H₂O + O₂ + O₃ + CO₂ goes through the conduct (6) into the wash tank (7) where the biological residuals molturated by the grinder (8) are. The wash tank (7) has a valve (12) from digasification to catalyzer. The residuals are exposed to a continuous bath with permanent recirculation of water with the chemical preparation, through the conduct (9), with a final spinning process, leaving the residuals sterilized.

## Description

### OBJECT OF THE INVENTION

This descriptive document refers to an application for a patent of invention which relates to a procedure, formula and equipment for the treatment and sterilization of biological, solid, liquid, iron-based metallic, non iron-based metallic, toxic and dangerous hospital residuals, based on a formula which, together with the appropriate equipment, will attain the aforementioned objective while offering doubtless advantages and improvements over currently-existing methods.

### FIELD OF APPLICATION OF THE INVENTION

This invention has its application within the industry which is dedicated to the manufacture of devices, procedures and equipment for the treatment and sterilization of biological wastes and residuals in general and particularly hospital residuals.

### BACKGROUND OF THE INVENTION

Currently known and existing techniques for the treatment of biological, solid, liquid, iron-based metallic, non iron-based metallic, toxic and dangerous hospital residuals are implemented with water vapor sterilizing apparatus or microwaves.

All these procedures have proved to be inadequate because the biological, solid, liquid, iron-based metallic, non iron-based metallic, toxic and dangerous hospital residuals, in addition to solid residuals such as:
- compresses,
- syringes,
- syringe plungers,
- glass tubes,
- glass plaques,
- catheters,
- i.v.s.
- bacterial culture plates,
- human appendages,
- needles,
- iron-based metals,
- non iron-based metals,
- various materials highly contaminated with bacteria and virus
- etc, etc.
are accompanied by semi-solid residuals tainted with purulence, blood, faeces, etc., all highly contaminating and extremely dangerous and, therefore, demanding not only of disinfection but of sterilization as well. If these biological, solid, liquid, iron-based metallic, non iron-based metallic, toxic and dangerous hospital residuals are not sterilized the surviving bacteria and virus will reproduce in the humidity and temperature of the disposal sites once the residuals are dumped, thus the sterilizers and microwaves merely cleanse and do not solve the real residual disposal problem. The residuals become an extremely dangerous source of disease contagion for people, animals, aquifers, and could even lead to epidemics of unsuspected magnitude.

The obvious solution to the currently existing problems would be the availability of a device, procedure, formula and equipment capable of overcoming these difficulties by providing a method of implementation together with the necessary devices and equipment capable of performing the adequate functions.

### DESCRIPTION OF THE INVENTION

The procedure, formula and equipment for the treatment and sterilization of biological, solid, liquid, iron-based metallic, non iron-based metallic, toxic and dangerous hospital residuals which this invention proposes constitutes in itself an application with which a total and absolute sterilization of biological, solid, liquid, iron-based metallic, non iron-based metallic, toxic and dangerous hospital residuals can be achieved, with the result that these products may be dumped into urban disposal sites without any risk whatsoever.

More specifically, the procedure, formula and equipment for the treatment and sterilization of biological, solid, liquid, iron-based metallic, non iron-based metallic, toxic and dangerous hospital residuals, object of this invention, begins with the introduction of the residuals into a grinder which consequently destroys all plastic components, syringes, syringe plungers, iron-based metals, non iron-based metals, test tubes, bacterial culture plates, glass materials, and any other existing solids and also manages to fragment and dissolve the semi-solid residuals present in gauzes and dressings so that they are ready to come in contact with the preparation H₂O + O₂ + O₃ + CO₂ and are also rendered non reusable once they are dumped.

Once the grinding/crushing and reduction of the biological, solid, liquid, iron-based metallic, non iron-based metallic, toxic and dangerous hospital residuals carried out by the grinder has concluded, the product obtained is put in a washer-spinner full of water. The residuals are washed, and the blood, purulent semi-solids, human appendages, etc. are diluted and left in conditions to receive the treatment which with a cryogenic tank, or with oxygen obtained from the atmosphere or from compressed and bottled air, with ozone or with carbon dioxide. These gases are made to pass through an ozone generating apparatus

The ozone plus oxygen, plus carbon dioxide in some cases, goes to a primary injector regulated by a pressurestat and these gases are mixed with the water provided by the pump. Next, in an initial stage the water and gases dissolved in it move on through a conduct to an absorption and digasification tank and from there, via another conduct, on to the wash and spin tank which contains the biological, toxic, and dangerous residuals. Then the entire device is turned on and H₂O + O₂ + O₃ + CO₂ or H₂O + O₂ + O₃ is recirculated. The residuals then undergo a continuous wash with permanent recirculation and a contact time between thirty seconds and forty-eight hours. Once the treatment time has elapsed, the biological, solid, liquid, iron-based metallic, non iron-based metallic, toxic, and dangerous hospital residuals are spun in order to remove any water remaining from the treatment. Given that the residuals are now sterilized, and thus similar to urban residuals, they are then removed from the machine and put into domestic or urban garbage containers.

In order to determine the antibacterial and antiviral activity of the chemical preparation employed in the treatment and sterilization of biological, solid, liquid, iron-based metallic, non iron-based metallic, toxic and dangerous hospital residuals that are the object of the invention, several different tests have been made, among them, the following studies:
- Study on the Antibacterial Activity of Ozone; issued by the Department of Microbiology,' Universidad Complutense de Madrid', School of Medicine.
- Study on the Antibacterial Activity of Ozonized Water; issued by the Department of Microbiology at the 'Universidad de Murcia', School of Medicine.
- Study on the Antibacterial Activity of Ozone; issued by the Health and Social Well-being Committee of the 'Junta de Comunidades de Castilla la Mancha'.
- Study on the Antibacterial Activity of Ozone; issued by the 'Servei de Salut, Hospital La Fe, Conselleria de Sanitat i Consum de la Generalitat Valenciana'.
- Study on Ozone in the Destruction of Spores; issued by the 'Laboratorios de Analisis Clinicos KOSLB, S.A.', Doctors J. Aznar, J. Báguera, M. Gobernado and V. Seco.

In essence, the procedure is a biological, solid, liquid, iron-base metallic, non iron-based metallic, toxic and dangerous hospital residuals sterilization system whose function is based on physical and chemical principles and the use of O₂ (oxygen), O₃ (ozone), CO₂ (carbon dioxide) and H₂O (water) and whose functional description, gas formulation and proportions, and mechanics consists of the formula O₂ (oxygen)+ O₃ (ozone) + H₂O (water) // O₂ (oxygen) + O₃ (ozone) + CO₂ (carbon dioxide) + H₂O (water) in the following proportions:
- O₂ (oxygen) from 0.01 liters per hour to 100 kilograms per hour.
- O₃ (ozone) from 0.01 grams per hour to 50 kilograms per hour.
- CO₂ (carbon dioxide) from 0.01 grams per hour to 50 kilograms per hour.
- H₂O (water) from 0.01 liters per hour to 1,000 cubic meters per hour.

Applying the process and chemical preparation previously described, the following results are obtained
- Applied Formula: H₂O + O₂ + O₃ + CO₂
- Water Volume: 500 liters per hour recirculated.
- Oxygen Volume: 1 cubic meter per hour.
- Nominal Ozone Production: 100 grams per hour.
- Concentration of Oxygen: 135 grams per cubic meter.
- Carbon Dioxide Volume: 0.2 cubic meters per hour.
- Temperature: 18 °C
- pH: 7.3

### DESCRIPTION OF THE DRAWINGS

To complete the description that is being given, and for a better understanding of the features of the invention, this descriptive document includes as part of the document, two pages of plans, in which the following has been illustrated (the scope of the drawing is for illustration only and should not be interpreted in a limiting sense):

Figure 1 shows a drawing which schematically represents the various elements of the system which is the object of the invention and which includes a procedure, formula and equipment for the treatment and sterilization of biological, solid, liquid, iron-base metallic, non iron-based metallic, toxic and dangerous hospital residuals.

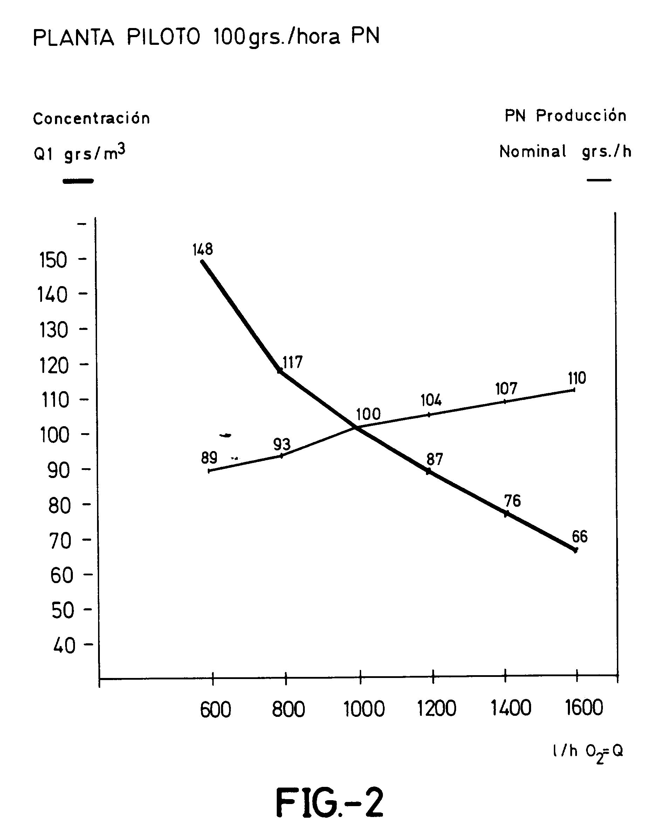

Figure 2 shows a graphic with the units of the mixture of gases, on a 100 basis, as implemented in a pilot equipment installation.

### PREFERRED REALIZATION OF THE INVENTION

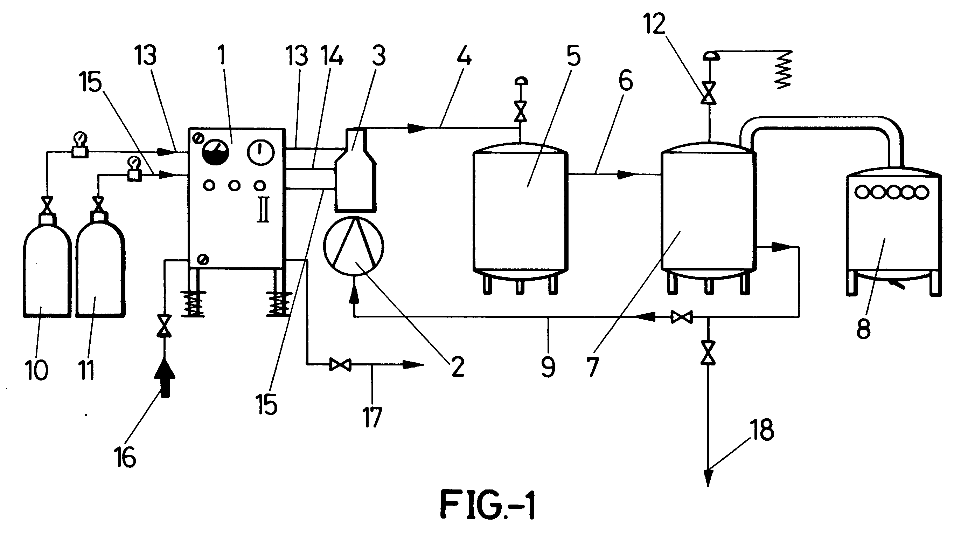

As Fig. 1 shows, it can be appreciated that the procedure, formula and equipment for the treatment and sterilization of the biological, solid, liquid, iron-base metallic, non iron-based metallic, toxic and dangerous hospital residuals that is proposed is made up of an Ozonizer (1), a Pump (2), an Injector (first stage) (3), a Conductor (4) for the H₂O + O₂ + O₃ + CO₂ mixture (first stage) and an Absorption and Digasification Tank (5) (second stage).

The invention has a homogenous flow (6) of H₂O + O₂ + O₃ + CO₂ and a BTDHR Wash Tank.

As it has been noted in the description of the invention, it also has a BTDHR Smash-Crush Grinder (8) as well as a Water Recirculation Conduct (9) for Recovery of the Chemical Preparation, a O₂ (oxygen) Bottle (10), a CO₂ (carbon dioxide) Bottle (11), a Valve between digasification to catalyzer (12), a O₂ (oxygen) + O₃ (ozone) conduct (13), a O₃ (ozone)+ O₂ (oxygen) conduct (14), a CO₂ (carbon dioxide) conduct (15), a Water Refrigeration Entrance (16), a Water Refrigeration Exit (17) and a Drain (18).

The function and application of the object of the invention for the treatment and sterilization of biological, solid, liquid, iron-based metallic, non iron-based metallic, toxic and dangerous hospital residuals is effected with compressed oxygen from bottles (10), with oxygen from a cryogenic tank or with oxygen obtained from atmospheric air or with compressed bottled air, with ozone (O₃), with carbon dioxide (CO₂) from bottles (11), whose characteristics have been previously described and which are made to pass through an ozone generating apparatus (1).

The ozone plus oxygen, plus carbon dioxide in some cases, and in others without carbon dioxide, goes to a primary injector (3) regulated by a pressurestat and is mixed with the water (6) provided by a pump (2). Next, this water (6), together with the gases dissolved in it is, in a fist stage, made to pass through a mixing system (4) and this mixing system itself moves the water into an absorption and digasification tank (5) and once these stages have been concluded, the water (H₂O), plus the gases, oxygen (O₂), ozone (O₃), and carbon dioxide (CO₂) are put into the washer (7) which contains the biological, solid, liquid, iron-based metallic, non iron-based metallic, toxic and dangerous hospital residuals from the crushing grinder (8) and then the aforementioned residuals are exposed to a continuous bath with permanent recirculation (9) with the water mix and a contact time which can vary from one second to forty-eight hours. Once this time has elapsed, the treatment water is separated and a centrifugation process is undergone.

Given that the residuals are now sterilized, and therefore can be incorporated into urban residuals, they are then removed from the machine and deposited in domestic and urban garbage containers, and the liquid solution also sterilized, is dumped into a sewage system drain.

We do not consider it necessary to make this description any more extensive, as any expert in the subject will understand the scope of the invention and the advantages that it provides.

The materials, form, size, and arrangement of the elements are susceptible to variation as long as this does not imply any alteration to the essence of the invention.

The terms that have been used to describe the invention in this document are to be interpreted throughout in their most ample sense and not in a limiting sense.

## Claims

1. Procedure, formula, and equipment for the treatment and sterilization of biological, solid, liquid, iron-based metallic, non iron-based metallic, toxic and dangerous hospital residuals, characterized essentially by the use of a chemical preparation according to the formula: O₂ (oxygen) + O₃ (ozone) + H₂O (water) // O₂ (oxygen) + O₃ (ozone) + CO₂ (carbon dioxide) + H₂O (water) and with the following proportions: O₂ (oxygen) from 0.01 liters per hour to 100 kiloliters per hour; O₃ (ozone) from 0.01 grams per hour to 50 kilograms per hour and H₂O (water) from 0.01 liters per hour to 1.000 cubic meters per hour. Previously, the BTDHR Wash Tank (7) has been filled and also the conduct containing the Pump (2), the Injector (3), the Conductor for the mixture (4), the Absorption and Digasification Tank (5), the homogenous flow (6) and the Water Recirculation Conduct (9) for the recovery of the chemical preparation. Then, the containers with BTDHR are introduced into the Grinder (8) and once they are reduced they are moved to the treatment Tank (7) where the Ozonizer (1) and the Pump (2) are started up maintaining during a period that can vary between 15 and 60 minutes the recirculation of water with H₂O + O₂ + O₃ + CO₂ or H₂O + O₂ + O₃ to obtain the dissolution of O₂ (oxygen) + O₃ (ozone) + CO₂ (carbon dioxide) or O₂ (oxygen) + O₃ (ozone) in the recirculation conduct, which is obtained by through the gases generated by the ozonizer (1) and through the injector (3) which receives the water from the pump (2). And once the gases O₂ (oxygen) + O₃ (ozone) have mixed up to a 20%, it passes through the conduct (4) on to the Absorption Tank (5), where the mixing of the gases O₂ (oxygen) + O₃ (ozone) reaches 80% and then through the conduct for homogenous flow (6) goes into the treatment Tank (7) where a 100% dissolution of the gases is achieved. There the period of contact with the BTDHR can vary between 15 and 60 minutes of treatment and a volume of carbon dioxide of 0.2 cubic meter per hour at a temperature of 18° and 7.3 pH.

2. Procedure, formula, and equipment description for the treatment and sterilization of biological, solid, liquid, iron-based metallic, non iron-based metallic, toxic and dangerous hospital residuals as per first claim distinguished because the units of the mixture of gases are described by the formula PN = Q x Q'/1000, being PN = Nominal Production; Q= Volume of O₂ (oxygen); Q' = Concentration of O₃ (ozone), on a 100 basis.

3. Procedure, formula, and equipment description for the treatment and sterilization of biological, solid, liquid, iron-based metallic, non iron-based metallic, toxic and dangerous hospital residuals characterized essentially by the first claim distinguished because the procedure with the chemical preparation is applied by means of a unit that, on the one hand, contains a solid smasher-grinder and from it, a washing tank, while the equipment itself, on the other hand, contains bottles with O₂ (oxygen) and CO₂ (carbon dioxide), and these pass through an ozonizing unit to an injector which shoots the gases (O₂ + CO₂ + O₃) through a mixing conduct on to an absorption tank with a water mix from which a homogenous flow conduct (H₂O + O₂ + O₃ + CO₂) connects to a washing tank with the biological, solid, liquid, iron-based metallic, non iron-based metallic, toxic and dangerous hospital residuals proceeding from a grinding smasher-crusher and which are exposed to a continuous bath with permanent recirculation of water with the chemical preparation with a contact time of one second to forty-eight hours. After the selected contact time has elapsed, the treatment water is separated and the materials are spun, after which, the residuals are left sterilized and, therefore, can be incorporated into urban residuals.
